# EUROPEAN PATENT APPLICATION

(11) **EP 4 567 026 A1**
(43) Date of publication of application: **11.06.2025**
(21) Application number: 24217627.9
(22) Date of filing: 05.12.2024
(51) Int. Cl.: C07D 213/53, C07D 239/26

(54) **SYNTHESIS OF BIS-OXIME HETEROCYCLES**

(30) Priority: 05.12.2023 US 202318529015
(71) Applicant: Southwest Research Institute, San Antonio, Texas 78238-5166 (US)
(72) Inventor: BLUMBERG, Shawn T., San Antonio, TX, 78201-5626 (US); DORSEY, Christopher L., San Marcos, TX, 78666 (US)
(74) Representative: Lucke, Andreas

(57) **Abstract**

A synthesis of bis-oxime heterocycles via reaction of 2,4-dimethyl pyridine or 2,4-dimethylpyrimidine in the presence of a phase transfer catalyst. Such bis-oxime heterocycles provide a more direct route to the synthesis of bis-quaternary pyridinium compounds and their analogues as nerve agent antidotes.

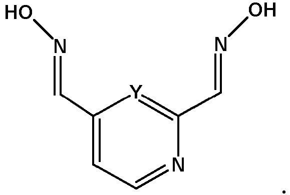

## Description

### GOVERNMENT SUPPORT CLAUSE

This invention was made with United States Government support under Contract No. OTA W1SQKN-16-9-1002, Project 24493 from the Defense Threat Reduction Agency/Department of Defense. The Government has certain rights in this invention.

### FIELD

The present invention provides a concise synthesis of bis-oxime heterocycles via reaction of 2,4-dimethyl pyridine or 2,4-dimethylpyrimidine in the presence of a phase transfer catalyst. Such bis-oxime heterocycles provide a more direct route to the synthesis of bis-quaternary pyridinium compounds and their analogs as nerve agent antidotes.

### BACKGROUND

Organophosphorous nerve agents (OPNA), utilized in chemical weapons and pesticides, irreversibly inhibit acetylcholinesterase enzyme (AChE) and cause an estimated 300,000 deaths per year worldwide. See, e.g., Eyer, P. et al, Toxicol. Rev. 2003 (22), 165-190. Bis-pyridinium oxime Hlo-7 dimethanesulfonate (DMS) (**1**) is among one of the most effective reactivators of OPNA-inhibited acetylcholinesterase (AChE).

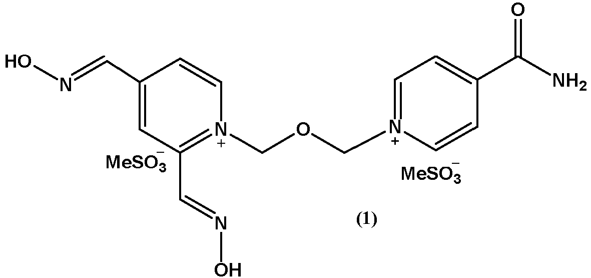

Hl07 DMS (**1**) can be synthesized by the unification of two fragments, the bisoxime compound (**2**), more specifically known as pyridine-2,4-aldoximne, and isonicotinamide (**3**), the former of which is not readily available.

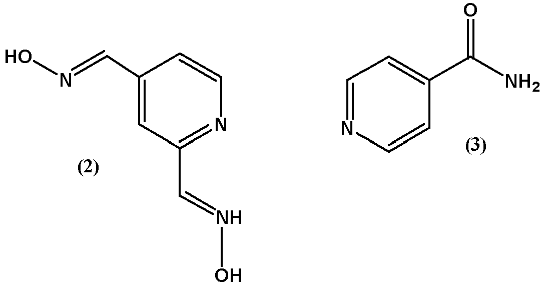

Current synthesis of the bisoxime heterocyclic compound (**2**) for use in organophosphorous antidote research has been a relatively laborious and time-consuming process that involves multiple synthetic transformations. As exemplified below, a bis-carboxylate is typically reduced, oxidized and condensed in hydroxylamine to form (**2**) in three steps. This requires the use of potentially pyrophoric reagents such as borane and the yield over the three steps is typically around 25.0 %.

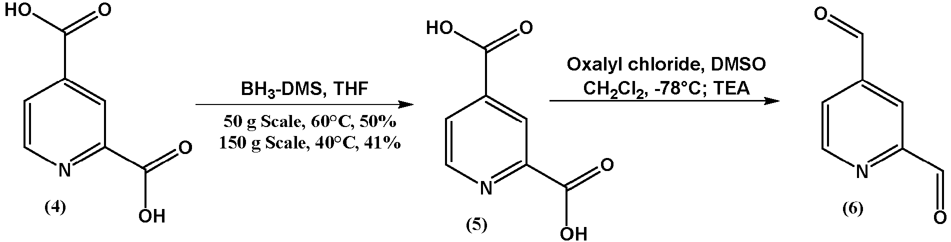

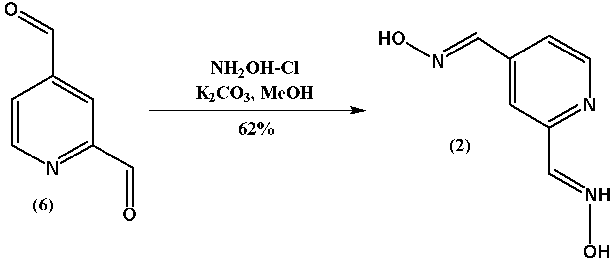

Another reported and relatively difficult route is to bis-oximes is illustrated below. As can be observed, a metal-halogen exchange, followed by quenching with DMF and subsequent condensation with hydroxylamine can also produce compound **(2**). However, this approach has the weakness of using pyrophoric organometallic reagents which require relatively specialized conditions to handle. Additionally, the starting materials for the above-described route (compound **4**) and the below route (compound **7**) are relatively expensive thereby dissuading such pathways for commercial preparation and limiting the diversity of the intermediates synthesized.

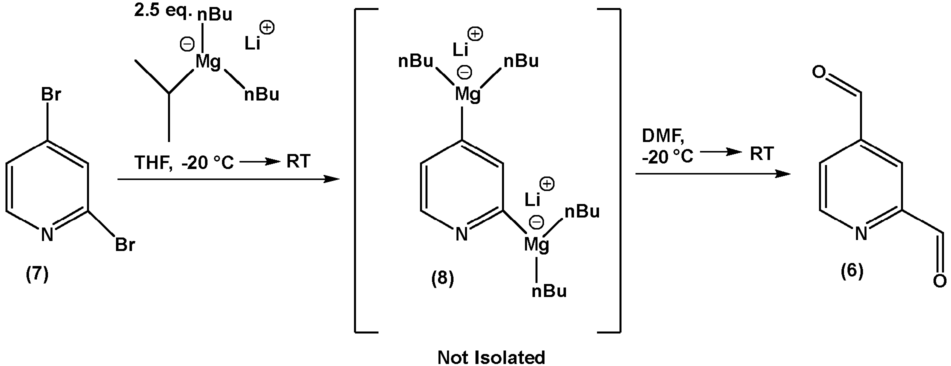

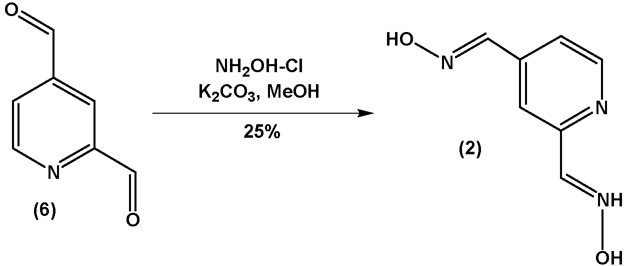

A need therefore remains to produce bisoxime heterocyclic type compounds such as 2,4-pyridinebisaldoxime or 2,4-pyrimidinebisaldoxime in more direct manner via the use of relatively less expensive reagents and at yields that will also be more amenable to commercial scale-up. Such an improved synthetic pathway to bisoxime compounds would also present a relatively more efficient route for the production of bis-quaternary pyridinium compounds or analogs thereof as nerve agent antidotes.

### SUMMARY

A method for preparing bis-oxime heterocycle compounds comprising:
a. converting 2,4-dimethylpyridine or 2,4-dimethylpyrimidine into a first mixture of the following metal salts, where said metal (**M**) is selected from sodium, potassium or lithium and Y is selected from carbon or nitrogen: 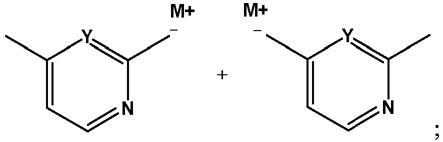
b. converting said first mixture of metal salts into the following mixture of nitroso compounds: 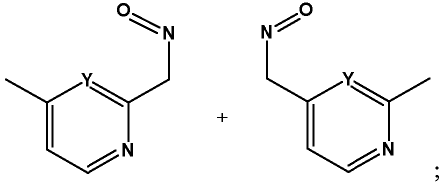
c. converting said mixture of nitroso compounds, in the presence of a phase transfer catalyst (PTC) and base, into the following transiently solubilized mono-oxime salt mixture: 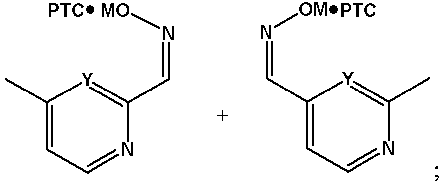
d. converting said transiently solubilized mono-oxime salt mixture into the following metal salt mixture: 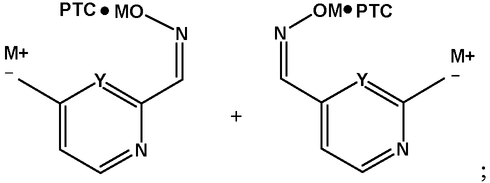
e. converting said metal salt mixture in (d) to a bis-oxime metal salt which is then protonated to form the following bis-oxime heterocycle compounds:

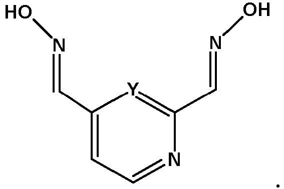

A method for preparing bis-oxime heterocyclic compounds comprising:
a. treating 2,4-dimethylpyridine or 2,4-dimethylpyrimidine with potassium tert-butoxide and forming a first mixture of the following potassium salts, where Y is selected from carbon or nitrogen: 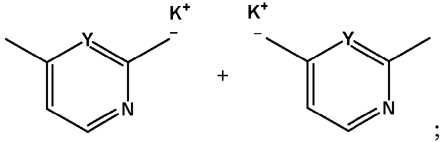
b. converting said first mixture of potassium salts into the following mixture of nitroso compounds: 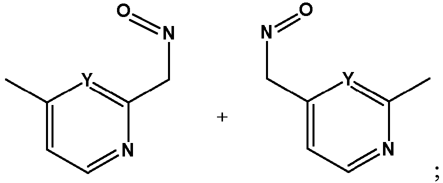
c. converting said mixture nitroso compounds, in the presence of a phase transfer catalyst (PTC) and potassium tert-butoxide, into the following transiently solubilized mono-oxime potassium salt mixture: 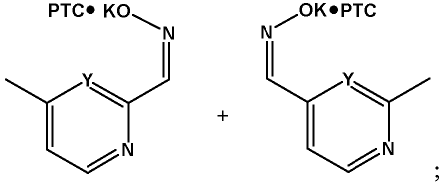
d. treating said transiently solubilized mono-oxime potassium salt mixture with potassium tert-butoxide and forming the following potassium salt mixture: 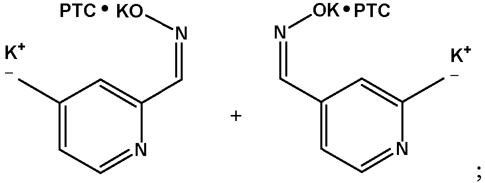
e. converting said potassium salt mixture in (d) to a bis-oxime potassium salt which is then protonated to form the following bis-oxime heterocycle compound:

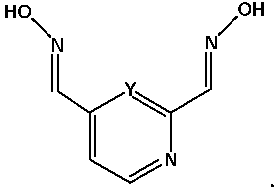

### DETAILED DESCRIPTION

To best describe the preferred embodiments of the present invention, it is worth an initial consideration of monoxime formation from methylpyridine derivatives with potassium tert-butyl nitrite (tBuOK) and tert-butyl nitrite (tBuONO). As illustrated below, treatment of 2,4-lutidine (8) (also described herein as 2,4-dimethylpyridine) with potassium tert-butoxide transiently deprotonates the 2,4-lutidine to form a mixture of 2,4-lutidine potassium salts (**9**).

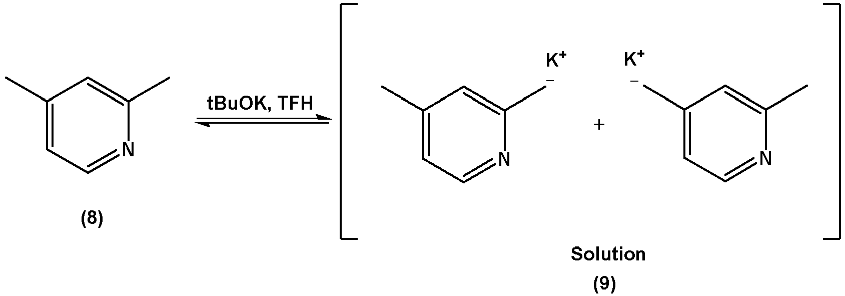

The mixture of 2,4-lutidine potassium salts (**9**) then reacts with tert-butyl nitrite to form a mixture of the intermediate 2,4-lutidine nitroso compounds (**10)**.

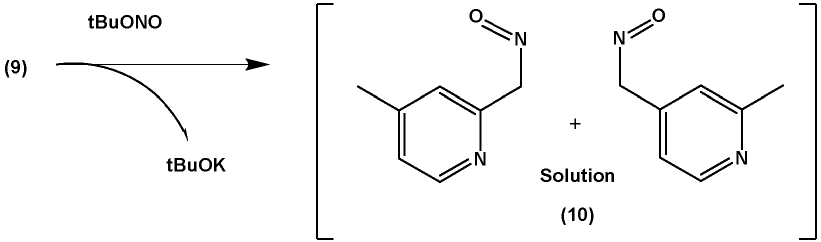

Compound **10** then is deprotonated to the oxime potassium salt mixture **11** which are very insoluble and precipitate out of solution (**12**) so no further reaction occurs resulting in the mono-oxime mixture **13**.

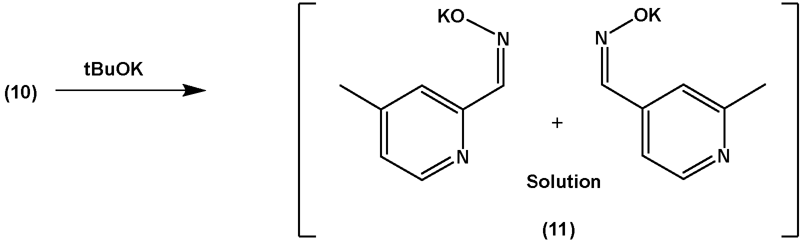

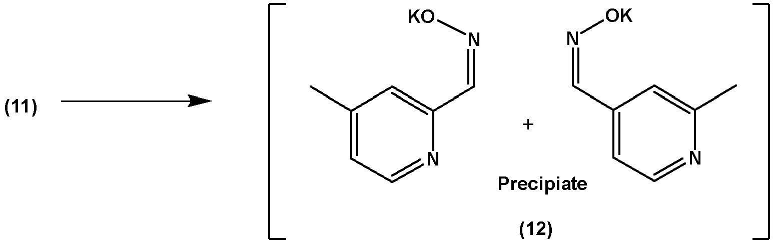

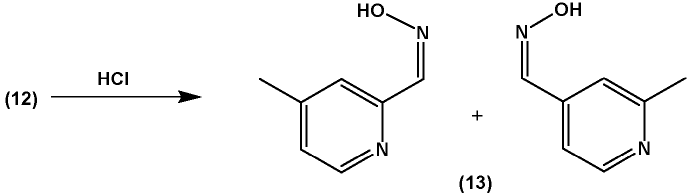

It has now been recognized that via use of a phase transfer catalyst (PTC) one can transiently solubilize the mono-oxime potassium salt (**11**) so that after a first methyl group in 2,4-lutidine is converted into an oxime potassium salt, the second methyl group in 2,4-lutidine is also converted into an oxime potassium salt, therefore resulting in bis-oxime formation. This route, among other things, may therefore provide for some of the following preferred advantages as compared to existing synthetic pathways to bis-oxime heterocycles: (a) use of relatively inexpensive feedstocks; (b) relatively less reaction steps; and/or (c) relatively higher yields.

The preferred route to the formation of bis-oxime heterocyclic compounds herein, such as 2,4-pyridinebisaldoxime or 2,4-pyrimidinebisaldoxime, begins again with 2,4-dimethylpyridine or 2,4-dimethylpyrimidine, which may be represented by the following: 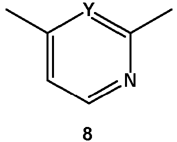 where Y is carbon or nitrogen. Accordingly, the 2,4-dimethylpyridine (Y=C) or 2,4-dimethylpyrimidine (Y=N) is treated with a metal tert-butoxide (tBuOM) in an organic solvent, preferably TFH. M may be selected from sodium, potassium or lithium.

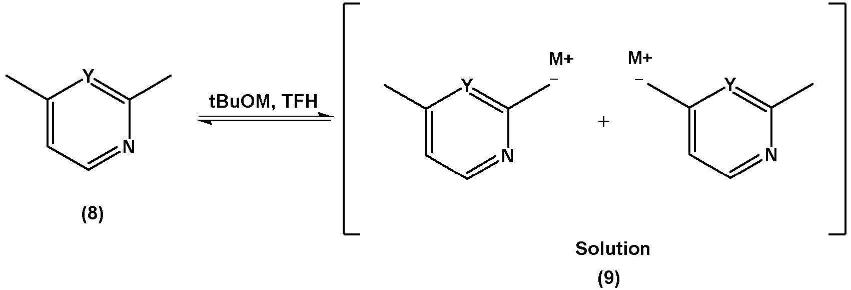

As previously noted, the mixture of metal salts (9) reacts with tert-butyl nitrite to again form a mixture of nitroso compounds (**10**):

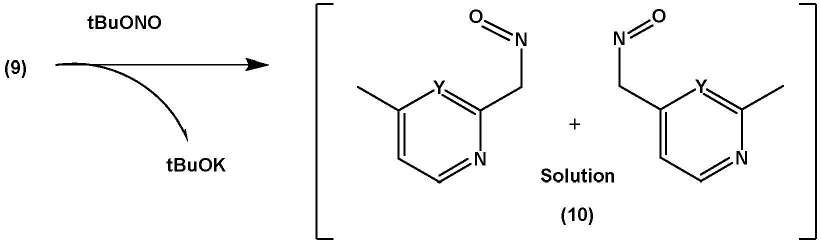

Compound **10** is then converted into an oxime salt mixture (**14**), preferably via treatment with a metal tert-butyl nitrite (tBuOM) wherein M= Na, K or Li, in the presence of an organic solvent, such as THF and in the presence of phase transfer catalyst (PTA), a preferred example of which is tris[2-(2-methoxyethoxy)ethyl] (TDA). This then provides for a transiently solubilized mono-oxime salt mixture (**14**) via chelation to the TDA phase transfer catalyst, which may be illustrated as follows:

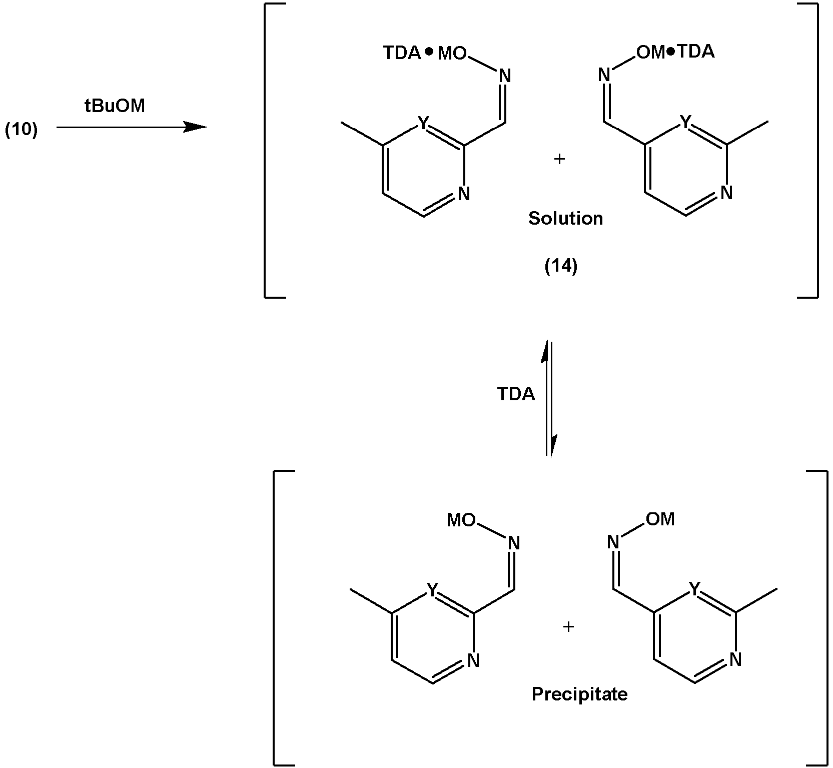

In the above, the chelation of the preferred phase transfer catalyst TDA to the metal (M), which again may be Na, K or Li, is illustrated by the indication: -OM•TDA. Other preferred phase transfer catalysts include but are not limited to 15-crown-5, 18-crown-6 or Polyethylene glycol (PEG). The mono-oxime salt mixture (**14**) that is therefore now solubilized by chelation of TDA to the metal (M) associated with the oxime oxygen atom will react with additional metal tert-butoxide (tBuOM) in solution where (M=Na, K or Li) and in a solvent such as THF, to form compound mixture **15**, where the second methyl group is now converted into a metal salt thereby providing the following mixture:

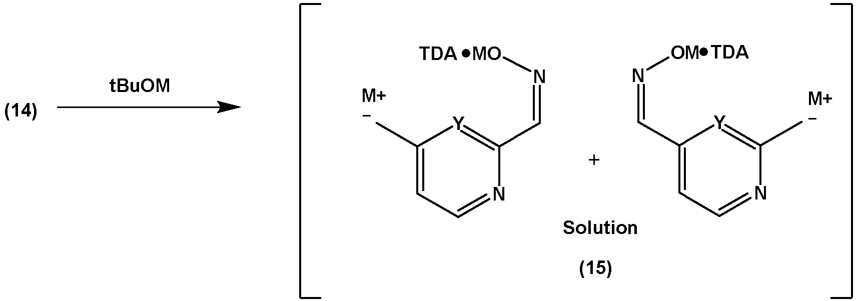

Compound mixture **15** is then treated with a metal (M) tert-butoxide (M=Na, K or Li) and tert-butyl nitrite in solution to form the bis-oxime metal salt **16** which is then preferably protonated by treatment with a mineral acid (e.g. HCl) to form pyridine-2,4-aldoximne (Y=C) or 2,4-pyrimidinebisaldoloxime (**17**) (Y=N).

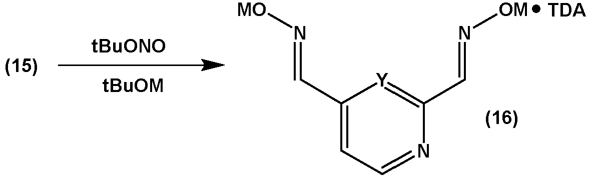

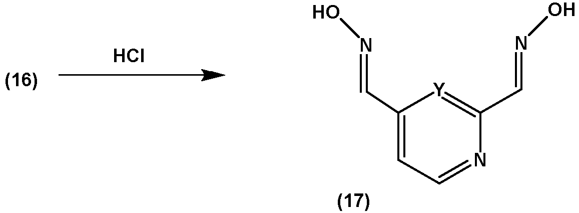

### Working Examples

### Preparation of 2,4-Pyridinebisaldoxime

To a 1 L nitrogen-purged flask was added potassium tert-butoxide (104.72g, 933 mmol), tert-butyl nitrite (44.4 mL, 38.5g, 374 mmol) and tris(2-(2-methoxyethoxy)ethyl)amine (29.9 mL, 30.19g, 93 mmol) and anhydrous 2-methyltetrahydrofuran (200 mL). A solution of 2,4-lutidine (10.79 mL, 109, 93 mmol) in anhydrous 2-methyltetrahydrofuran (100 mL) was added dropwise making sure to keep the temperature below 40 °C. After addition was complete, the reaction was allowed to stir at RT overnight. The reaction was quenched with water (100 mL) and adjusted to pH 2-3 by the slow addition of 2M HCl and the organic layer separated and washed with 50 mL of 2M HCl. The aqueous layers were combined and neutralized to pH 7-8 with potassium carbonate and extracted with 2-methyltetrahydrofuran (3 x 100 mL). The organic layers were combined, dried over sodium sulfate, filtered and solvent removed under reduced pressure. The crude solid was then triturated with dichloromethane (150 mL) for 3 hrs. and filtered to yield the desired 2,4-pyridinebisaldoxime (10.59 g, 68.7% yield) as an off white solid.

### Preparation of 2,4-pyrimidinebisaldoxime

To a 250 mL nitrogen-purged flask was added potassium tert-butoxide (31.1 g, 277 mmol), tert-butyl nitrite (13.2 mL, 110.8 mmol), tris(2-(2-methoxyethoxy)ethyl)amine (8.86 mL, 27.7 mmol), and anhydrous 2-methyltetrahydrofuran (90 mL). A solution of 2,4-dimethylpyrimidine (3.0 g, 27.7 mmol) in anhydrous 2-methyltetrahydrofuran (15 mL) was added dropwise making sure to keep the temperature below 40 °C. After addition was complete, the reaction was allowed to stir at RT overnight. The reaction was quenched with water (30 mL) and adjusted to pH 2-3 by the slow addition of 2M HCl. The organic layer was separated and washed with 20 mL of 2M HCl. The aqueous layers were combined and neutralized to pH 7-8 with potassium carbonate and extracted with 2-methyltetrahydrofuran (3 x 30 mL). The organic layers were combined, dried over sodium sulfate, filtered, and concentrated under reduced pressure. The crude solid was then triturated with dichloromethane (15 mL) for 1h and filtered to yield the desired 2,4-pyrimidinebisaldoxime (1.54 g, 33.5 % yield) as a yellow solid.

As may now be appreciated, the present invention relates to a method for producing the bis-oxime heterocyclic compounds 2,4-pyridinebisaldoxime or 2,4-pyrimidinebisaldoxime from a relatively inexpensive feedstock of 2,4-dimethylpyridine or 2,4-dimethylpirimidine by conversion into first mixture of metal salts wherein a first methyl group on said 2,4-pyridinebisaldoxime or 2,4-pyrimidinebisaldoxime is converted a metal salt mixture (**9**). Such mixture of metal salts is then converted into a mixture of nitroso compounds (**10**) which are then converted into an oxime salt mixture in the presence of a phase transfer catalyst to provide a transiently solubilized mono-oxime salt mixture (**14**). The transiently solubilized mono-oxime salt mixture (**14**) then allows for the second methyl group of the 2,4-pyridinebisaldoxime or 2,4-pyrimidinebisaldoxime to form into a metal salt (**15**) which is then converted to an oxime to provide a bis-oxime potassium salt which is then converted to form 2,4-pyridinebisaldoxime or 2,4-pyrimidinebisaldoxime (**17**). The overall yield of pyridine-2,4-bisaldoxime is at least 60% and preferably in the range of 60.0% to 70.0%. The overall yield of 2,4-pyrimidinebisaldoxime is at least 30% and preferably in the range of 30.0 to 40.0 %.

## Claims

1. A method for preparing bis-oxime heterocycle compounds comprising:
a. converting 2,4-dimethylprydine or 2,4-dimethylpyrimidine into a first mixture of the following metal salts, where said metal (M) is selected from sodium, potassium or lithium and Y is selected from carbon or nitrogen: 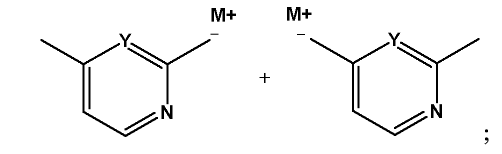
b. converting said first mixture of metal salts into the following mixture of nitroso compounds: 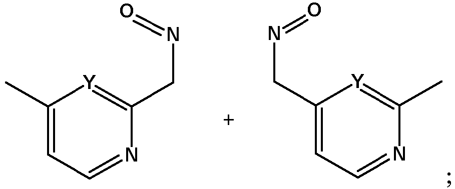
c. converting said mixture of nitroso compounds, in the presence of a phase transfer catalyst (PTC) and base, into the following transiently solubilized mono-oxime salt mixture: 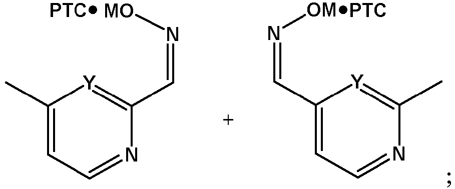
d. converting said transiently solubilized mono-oxime salt mixture into the following metal salt mixture: 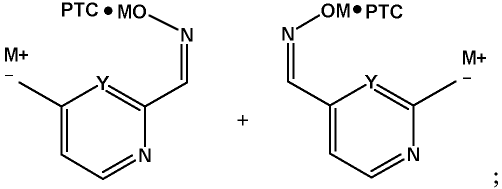
e. converting said metal salt mixture in (d) to a bis-oxime metal salt which is then protonated to form the following bis-oxime heterocycle compounds:

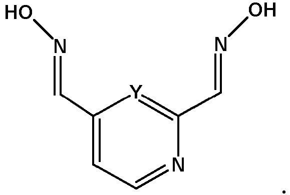

2. The method of claim 1, wherein said phase transfer catalyst comprises tris[2-(2-methoxyethoxy)ethyl]amine (TDA).

3. The method of claim 1, wherein said phase transfer catalyst comprises 15-crown-5 or 18-crown-6.

4. The method of claim 1 wherein said phase transfer catalyst comprises polyethylene glycol).

5. The method of claim 1, wherein 2,4-dimethylpyridine or 2,4-dimethylpyrimidine is converted into said first mixture of metal salts by treatment with a metal tert-butoxide (tBuOM) where M is sodium, potassium or lithium.

6. The method of claim 1, wherein said mixture of metal salts is converted into said mixture of nitroso compounds by treatment with tert-butyl nitrite.

7. The method of claim 1, wherein said mixture nitroso compounds are converted in the presence of said phase transfer catalyst into said transiently solubilized mono-oxime salt mixture by treatment with a metal tert-butyl nitrite (tBuOM) where M=Na, K or Li.

8. The method of claim 1, wherein said bis-oxime heterocyclic compound formed in (e) is pyridine-2,4-bisaldoxime at a yield of at least 60%, or wherein said bis-oxime heterocyclic compound formed in (e) is 2,4-pyrimidinebisaldoxime at a yield of at least 30%.

9. A method for preparing a bis-oxime heterocyclic compound comprising:
a. treating 2,4-dimethylpyridine or 2,4-dimethylpyrimidine with potassium tert-butoxide and forming a first mixture of the following potassium salts, where Y is selected from carbon or nitrogen: 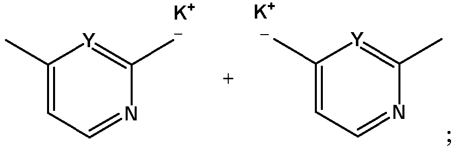
b. converting said first mixture of potassium salts into the following mixture of nitroso compounds: 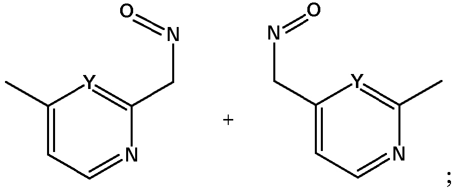
c. converting said mixture nitroso compounds, in the presence of a phase transfer catalyst (PTC) and potassium tert-butoxide, into the following transiently solubilized mono-oxime potassium salt mixture: 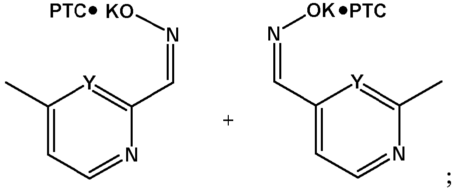
d. treating said transiently solubilized mono-oxime potassium salt mixture with potassium tert-butoxide and forming the following potassium salt mixture: 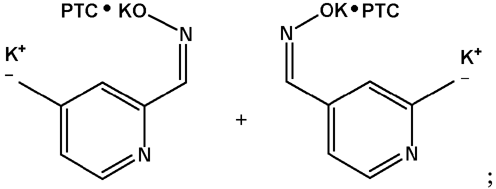
e. converting said potassium salt mixture in (d) to a bis-oxime potassium salt which is then protonated to form the following bis-oxime heterocycle compound:

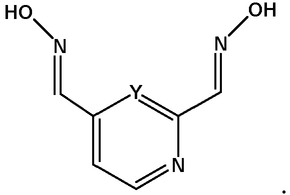

10. The method of claim 9, wherein said phase transfer catalyst comprises tris[2-(2-methoxyethoxy)ethyl]amine (TDA).

11. The method of claim 9, wherein said phase transfer catalyst comprises 15-crown-5 or 18-crown-6.

12. The method of claim 9, wherein said phase transfer catalyst comprises polyethylene glycol).

13. The method of claim 9, wherein 2,4-dimethylpyridine or 2,4-dimethylpyrimidine is converted into said first mixture of potassium salts by treatment with a metal tert-butoxide (tBuOM) where M is potassium.

14. The method of claim 9, wherein said mixture of nitroso compounds are converted in the presence of said phase transfer catalyst into said transiently solubilized mono-oxime salt mixture by treatment with a metal tert-butyl nitrite (tBuOM) where M=K.

15. The method of claim 9, wherein said bis-oxime heterocyclic compound formed in (e) is pyridine-2,4-bisaldoxime at a yield of at least 60%, or wherein said bis-oxime heterocyclic compound formed in (e) is 2,4-pyrimidinebisaldoxime at a yield of at least 30%.
